# EUROPEAN PATENT APPLICATION

(11) **EP 1 245 955 A1**
(43) Date of publication of application: **02.10.2002**
(21) Application number: 00985915.8
(22) Date of filing: 27.12.2000
(51) Int. Cl.: G01N 33/53, G01N 33/50, C12N 15/19, C07K 7/08

(54) **CYTOKINE-LIKE PEPTIDE**

(30) Priority: 27.12.1999 JP 36999099
(71) Applicant: TORAY INDUSTRIES, INC., Tokyo 103-8666 (JP)
(72) Inventor: SATO, Atsushi, Kamakura-shi, Kanagawa 248-0034 (JP); SONE, Saburo, Yokohama-shi, Kanagawa 244-0817 (JP)
(74) Representative: Kador & Partner
(86) International application number: JP0009278
(87) International publication number: WO01048478

(57) **Abstract**

The present invention relates to: a method for screening a cytokine-like peptide, comprising searching for a peptide which binds to an antibody having an activity of neutralizing a cytokine and which is capable of expressing a biological activity of the cytokine from among peptides not confirmed as having the biological activity of the cytokine; a cytokine-like peptide obtained by the screening method; and a medicine comprising, as an active ingredient, the peptide.

SEQUENCE LISTING

<110> TORAY INDUSTRIES, INC.

<120> CYTOKINE-LIKE PEPTIDE

<130> PH-1101-PCT

<150> JP99/369990
<151> 1999-12-27

<160> 1

<210> 1
<211> 15
<212> PRT
<213> Artificial Sequence

<220>
<223> Designed peptide based on the DNA sequence of phage binding to
monoclonal antibody YSB-2

<400> 1
Ser Val Gln Ala Arg Trp Glu Ala Ala Phe Asp Leu Asp Leu Tyr
1               5                   10                  15

## Description

### TECHNICAL FIELD

The present invention relates to a peptide which binds to a neutralizing antibody for a cytokine and which is capable of expressing a biological activity of the cytokine, and more particularly to a peptide which is usable in the treatment of viral diseases and the like and a method for screening the peptide.

### BACKGROUND ART

Proliferation and differentiation of animal cells are controlled by various intercellular signal transduction molecules. Proteinous molecules, which are responsible for this signal transduction, include so-called growth factors, i.e., a group of proteins including proteins referred to as a lymphokine, a monokine, an interferon, a chemokine, a hemopoietic factor, a neurotrophic factor and the like.

"Cytokine" is a generic term for these proteinous signal transduction molecules. A cytokine exhibits a variety of physiological activities. Physiological activities of the cytokine include, for example, an antiviral activity or antitumor activity of the interferon (IFN), formation of a granulocyte colony by a granulocyte colony stimulator (G-CSF), generation of erythrocytes by erythropoietin (EPO), and proliferation of megakaryocyte by thrombopoietin (TPO). Many of these cytokines are used in the actual treatment of diseases by making use of their physiological activities. Since these cytokines are high molecular weight proteins, they are produced by culturing a large amount of cells producing cytokines or culturing a large amount of cells in which gene recombination was carried out for producing cytokines or microorganisms. Further, in order to obtain a cytokine of a single molecular species, a complicated operation is required in which the subject cytokine is purified from a cell culture supernatant or a solution containing crushed cells or microorganisms. Because the purified cytokine thus obtained is a high molecular weight protein, in general, its stability is not high. Therefore, in order to overcome drawbacks such as the complicated steps in producing and purifying the cytokines or lack of stability in the subject substances, a method in which the physiological activities of cytokines are mimicked by a low molecular weight peptide is effective. The low molecular weight peptide can be prepared by chemical synthesis and has excellent stability. There are reports, as examples thereof, on a low molecular weight peptide that mimics erythropoietin (EPO) (Wrighton, N. C. et al., Science, 273, 458-463 (1996)) or thrombopoietin (TPO) (Cwirla, S. E. et al., Science, 276, 1696-1699 (1997); Kimura, T. et al., J. Biochem., 122, 1046-1051 (1997)) using a phage peptide library technique.

In both of these methods, however, the phage peptide library is screened using a cytokine receptor as a target molecule, thereby obtaining the subject low molecular weight peptide. In this case, a drawback exists in that the preparation of the cytokine receptor is complicated. If a method for newly producing a low molecular weight peptide that mimics a cytokine without the use of a receptor molecule can be developed, it is considered that the versatility will be high.

### DISCLOSURE OF THE INVENTION

The object of the present invention is to provide a method for newly producing a low molecular weight peptide that mimics a cytokine, which has overcome the above drawbacks. More specifically, the object of the present invention is to provide a method for newly producing a low molecular weight peptide that mimics a cytokine by selecting the sequence binding to the neutralizing antibody for the cytokine from random amino acid sequences.

The present invention includes the following inventions.
(1) A method for screening a cytokine-like peptide, comprising searching for a peptide which binds to an antibody having an activity of neutralizing a cytokine and which is capable of expressing a biological activity of the cytokine from among peptides not confirmed as having the biological activity of the cytokine.
(2) The screening method according to (1) above, wherein the cytokine is an interferon.
(3) A cytokine-like peptide, which is obtained by the screening method according to (1) or (2) above.
(4) A cytokine-like peptide, which contains an amino acid sequence derived from the amino acid sequence of the cytokine-like peptide according to (3) above by deletion, substitution, insertion or addition, or modification of at least one amino acid residue and which is capable of expressing the biological activity of the cytokine.
(5) The cytokine-like peptide according to (4) above, wherein the amino acid sequence of the cytokine-like peptide is the amino acid sequence shown in SEQ ID NO: 1 of the Sequence Listing and the cytokine is an interferon.
(6) A cytokine-like peptide capable of expressing a biological activity of an interferon, wherein the amino acid sequence of the cytokine-like peptide contains the amino acid sequence shown in SEQ ID NO: 1 of the Sequence Listing.
(7) A method for producing a cytokine-like peptide, comprising obtaining a peptide capable of expressing a biological activity of a cytokine, wherein the cytokine-like peptide obtained by the screening method according to (1) or (2) above is employed as a leading compound and deletion, substitution, insertion or addition, or modification of at least one amino acid residue is applied thereto.
(8) A medicine comprising, as an active ingredient, the cytokine-like peptide according to any one of (3) to (6) above.

The peptide of the present invention has similar biological activities as the cytokine. The term "cytokine" used herein includes, for example, an interferon (for example, α, β, and γ), a growth factor, a lymphokine, a monokine, a chemokine, a hemopoietic factor, a neurotrophic factor, a tumor necrosis factor, and a lymphotoxin.

In the present invention, a monoclonal antibody is preferred as an antibody having an activity of neutralizing a cytokine, although a polyclonal antibody can also be used.

In the preparation of a monoclonal antibody for the cytokine, a general method is a method using a hybridoma that has been developed by Kohler and Milstein (Kohler and Milstein, Nature, 256, 495-497 (1975)). An antibody-producing hybridoma is produced through *in vitro* cell fusion between antibody-producing cells incapable of autoproliferation (B lymphocytes in spleen and lymph node) and myeloma cells which proliferate infinitely. Thereafter, the detection of antibodies in the hybridoma culture supernatant and cloning of cells are repeated using a suitable assay system, thereby obtaining homogenous antibody-producing hybridoma (a separate volume of Experimental Medicine, A New Handbook for Genetic Engineering, p. 162-165, YODOSHA CO., LTD.). The obtained homogenous antibody-producing hybridoma is subjected to mass culture and the monoclonal antibody secreted in the culture supernatant is easily purified with Protein A column in the case of IgG. Whether the monoclonal antibody thus established is capable of neutralizing the activity of a cytokine or not can be determined by various methods depending on the types of cytokine. For example, in the case of an interferon, a bioassay method can be employed in which human amnion-derived FL cells are combined with sindbis viruses or vesicular stomatitis viruses (VSV). This method is reported by, for example, Kawade et al. (Kawade. Y. and Watanabe, Y. J., IFN Res. 4, 571-584 (1984)).

Methods for obtaining the peptide of the present invention include, for example, a peptide library technique described below. Further, the peptide library technique can be divided into a method in which bacteriophage is used and a method in which a library is constructed through chemical synthesis.

A method for constructing a phage random peptide library can be carried out by, for example, connecting a synthetic gene having a random sequence to genes for a coat protein of M13 phage (for example, gene III protein or IIIV protein). As such a method, a method described in, for example, Science, 249, 386 (1990) or Proc. Natl. Acad. Sci. USA, 87, 6378 (1990) can be employed. The size of genes to be inserted is not particularly limited as long as the expressed peptide is stable. However, in order for the produced library to cover a larger number of random sequences and to be capable of binding to the target molecule, a size of 6 to 15 amino acids is preferred. In order to select the phage for binding to the subject monoclonal antibody, the purified monoclonal antibody is immobilized on a column or a microtiter plate directly or through an anti-IgG antibody etc., thereby allowing the library to contact. Thereafter, non-binding phage is washed away through a lavage operation. After washing, a bound phage is eluted with an acid. After neutralization, the eluted phage is infected with *Escherichia coli* and amplified. By repeating this operation (panning) three or four times, phage having affinity with a monoclonal antibody is concentrated. In order to obtain a single clone, phage is reinfected with *Escherichia coli* and a single colony is formed on an agar medium containing an antibiotic. After culturing an individual colony in a liquid medium, phage in the supernatant is concentrated by precipitation with the aid of polyethylene glycol and the like. If the nucleotide sequence thereof is determined, the peptide structure can be known.

In addition to the above method using phages, a peptide library having random amino acid sequences can also be produced through chemical synthesis. The methods include a method using beads (Nature, 354, 82 (1991)), a liquid-phase focusing method (Nature, 354, 84 (1991)), and a microplate method (Science, 251, 767 (1991)).

Methods for mass-producing peptides having sequences obtained from the library include a method for artificially synthesizing peptides and a method utilizing a gene recombination technique to express in, for example, *Escherichia coli*, yeast, insect cells, and animal cells.

A method for artificially synthesizing peptides can be easily carried out using a general method for synthesizing peptides. For example, it can be simply carried out by a solid-phase synthesis method, and a variant can be easily prepared in which a deletion, substitution, insertion, or addition is applied to the subject sequence (a separate volume of Cell Engineering, Experimental Protocol for anti-peptide antibody, p. 26- p. 46, SHUJUNSHA). Modification may also be applied, such as performing introduction of a non-naturally occurring amino acid, chemical modification of each amino acid residue or introduction of a cysteine residue, to cyclize inside the molecule, thereby stabilizing the structure.

In the case where a gene recombination technique is employed, determination of the DNA sequence from the obtained amino acid sequence in accordance with the codon usage (see Molecular Cloning, Appendix D1, Maniatis et al.; Cold Spring Harbor Laboratory, 1989) and introduction into the host cell are technically established. Further, introduction of variation into a nucleotide sequence enables conversion of the amino acid into another residue. For example, when expressed in *Escherichia coli*, the obtained DNA sequence is bound to a promoter sequence, for example, tryptophan synthetase operon (Trp) or a lactose operon (lac) promoter and a ribosome binding sequence, for example, Shine-Dalgarno (SD) sequence, or a recognition site for a transcription termination factor is preferably added. Methods for introducing the prepared expression vector into *Escherichia coli* usable herein include, for example, a method described in Molecular Cloning (Maniatis et al.; Cold Spring Harbor Laboratory, 1989). In the method for purifying the expression product, for example, various chromatography techniques can be employed.

Methods for inspecting whether the obtained peptide has a physiological activity of the cytokine or not vary depending on the type of cytokines. For example, in the case of an interferon it can be evaluated by determining the antiviral activity. More specifically, evaluation is carried out by employing a bioassay method in which human amnion-derived FL cells are combined with sindbis viruses or vesicular stomatitis viruses (VSV) (Armstrong, J. A., Methods in Enzymology, 78, 381-387 (1981)).

A peptide confirmed as having cytokine-like activity in accordance with the screening method of the present invention is employed as a leading compound and deletion, substitution, insertion or addition, or modification of at least one amino acid residue is applied thereto, thereby obtaining a peptide capable of expressing the physiological activity of the cytokine. Thus, an analogous cytokine-like peptide can be produced. Whether or not this peptide has a physiological activity of the cytokine can be examined in the same manner as described above.

The present invention also provides a cytokine-like peptide obtained by the screening method and a medicine comprising, as an active ingredient, the peptide. The phrase "peptide obtained by the screening method" used herein refers to a peptide for which the cytokine-like activity is actually confirmed by the screening method.

The number of amino acid residues of the peptide according to the present invention is generally 6 to 40, and preferably 6 to 15.

Examples of the peptides of the present invention include a peptide which contains the amino acid sequence shown in SEQ ID NO: 1 of the Sequence Listing or an amino acid sequence obtained from the amino acid sequence shown in SEQ ID NO: 1 by deletion, substitution, insertion or addition, or modification of at least one amino acid residue and which is capable of expressing a biological activity of an interferon. The peptide has an antiviral activity, an antitumor activity, or an immunoregulatory activity.

The cytokine-like peptide of the present invention can be orally or parenterally administered as is or as a pharmaceutical composition mixed with, for example, a conventional pharmaceutically acceptable carrier or excipient.

The formulation for the oral administration specifically includes tablets, pills, capsules, granules, fine granules, powders, syrups, emulsions, and suspensions. The formulation is produced by a conventional method and comprises a carrier or an excipient commonly used in the field of medicine. Examples of a carrier or excipient for tablets include lactose, maltose, sucrose, starch, and magnesium stearate.

The formulation for the parenteral administration includes, for example, eye drops, ointments, injections, fomentations, suppositories, transnasal preparations, transpulmonary preparations, transdermal preparations, and local sustained release preparations. A liquid preparation can be prepared by a conventional method, for example, one in which a cytokine-like peptide is generally dissolved in a sterile aqueous solution that is used in injections and further emulsified, and the peptide is encapsulated in liposome. A solid preparation can be prepared by a conventional method, for example, one in which mannitol, trehalose, sorbitol, lactose, glucose or the like is added to the cytokine-like peptide as an excipient and, in that state, freeze-dried. Further, this can be used in a powdered state. These powders can be used in a solidified state by mixing with a polylactic acid, glycolic acid, or the like. A gelling agent can be prepared by a conventional method, for example, one in which the cytokine-like peptide is dissolved in a thickener such as glycerin, polyethylene glycol, methyl cellulose, carboxymethyl cellulose, hyaluronic acid, and chondroitin or polysaccharides.

In any of these preparations, human serum albumin, human immunoglobulin, α₂ macroglobulin, an amino acid or the like can be added as a stabilizer. Also, alcohol, sugar alcohol, ionic surfactant, nonionic surfactant or the like can be added as a dispersant or absorption enhancer to the extent that the physiological activity of the cytokine-like peptide is not deteriorated. A trace of metal or a salt of organic acid can be added if necessary.

The cytokine-like peptide of the present invention can be prepared into the above-described formulations and used in the treatment of various diseases. In the case of a peptide capable of expressing the biological activity of an interferon, for example, it can be used in the treatment of chronic active hepatitis B, chronic hepatitis C, and other viral diseases, various malignant neoplasms such as gliosarcoma, medulloblast, astroglioma, and cutaneous malignant melanoma, and autoimmune diseases such as multiple sclerosis and the like. Further, it can be used in the treatment of diseases involving neovascularization, for example, inflammatory diseases such as rheumatic arthritis and proriasis, diabetic retinopathy, prematurity retinopathy, neovascular glaucoma, Stevens-Johnson syndrome and analogous diseases thereof, ocular pemphigus and analogous diseases thereof, eye diseases such as corneal chemical injury and trachoma, and cancers (for example, breast cancer, prostate cancer, malignant melanoma, renal cancer, brain tumor, and Kaposi's sarcoma).

In the medicine of the present invention, the dosage of peptide as an active ingredient varies depending of the activity of the peptide, the age and the weight of the patient, and the type or extent of diseases. In the oral administration, the dosage is generally 0.001 to 1,000 mg/kg, based on the weight of the patient, per day and, in the intravenous, intramuscular, or subcutaneous administration, the dosage is generally 0.001 to 1,000 mg/kg, based on the weight of the patient, per day. The frequency of administration is generally 1 to 3 times per day in the oral administration and 1 to 2 times per day in the case of injection.

The secondary structure of the peptide can be found out by measuring the circular dichroism (CD). In general, a low molecular weight peptide (of a length of about 15 amino acid residues) often exhibits an irregular structure without forming a specific secondary structure. If the peptide has a secondary structure, the structure in a solution can be analyzed using NMR. Further, a stable low molecular weight compound can be designed through chemical synthesis using examples from the peptide structure.

### BRIEF DESCRIPTION OF THE DRAWINGS

Fig. 1 shows the result of examination by ELISA on the reactivity of the phages obtained through screening. In the drawing, the horizontal axis represents names of the phage clones (phage 6 is a clone having a sequence shown in SEQ ID NO: 1 in the Sequence Listing and fuse 2 represents a control phage having no random sequence) and the vertical axis represents the absorbance at 450 nm.
Fig. 2 shows the amino acid sequences of the obtained phage clones. An amino acid residue is represented by a single character.
Fig. 3 shows the result of measurement of the antiviral activity of the synthetic peptides. The peptides synthesized in accordance with the sequences shown in Fig. 2 were designated as peptides SYR1, SYR2, and SYR6 in accordance with the sequences of phage 1, phage 2, and phage 6. Two peaks were detected in SYR6 when purified by a reverse phase HPLC and, thus, the peptides at each peak, i.e., SYR6A and SYR6B were separately measured. The activity was recognized in SYR6 and there was no difference in the activity of SYR6A and SYR6B. As a control, the result of interferon β is also presented.
Fig. 4 shows the result of measurement of the antiviral activity of the synthetic peptide SYR6. It was examined by a bioassay method using human amnion-derived FL cells and vesicular stomatitis viruses (VSV). As a control, the result of interferon β is also presented.
Fig. 5 shows the result of measurement of the antiviral activity of synthetic peptide SYR6 in the presence of various interferon neutralizing antibodies. As a control, the result of interferon β is also presented.
Fig. 6 shows a CD spectrum of peptide SYR6. A negative maximum was observed at around 213 nm and peptide SYR6 is considered to be mainly constructed by β-sheet.
Fig. 7 shows the antiviral activity of SYR6 peptide substituted with an alanine residue in the upper part, and amino acid sequences in the lower part.
Fig. 8 shows the result of a solid-phase ligand binding assay using a soluble interferon receptor. Binding between interferon β and a soluble interferon receptor was observed in a concentration-dependent manner.
Fig. 9 shows the result of analysis on the binding of peptides SYR1, SYR2, and SYR6 to a soluble interferon receptor using a competitive binding assay. Regarding SYR1 and SYR2, binding was not inhibited even with the addition of 100 µM thereof, however, with the addition of 25 µM to 100 µM of SYR6 the binding was inhibited in a concentration-dependent manner.

This specification includes part or all of the contents disclosed in the specification of Japanese Patent Application No. 11-369990, which is the priority document of the present invention.

### BEST MODE FOR CARRYING OUT THE INVENTION

The present invention will be hereinafter described in more detail with reference to examples although the present invention is not limited to these examples.

### Example 1: Preparation of YSB-2 which is a human interferon β neutralizing antibody.

Hybridoma producing YSB-2, which is a monoclonal antibody having an activity of neutralizing human interferon β (Sugi, M. et al., Hybridoma, 6, 313-320 (1987)), was cultured in a serum-free medium (Hybridoma SFM, Gibco BRL). After culturing for about one week, cells were precipitated by centrifugation and a culture supernatant containing an antibody was obtained. Debris of the cell was removed using a 0.45 µM filter and the culture supernatant was then purified using Protein A column (Amersham Pharmacia Biotech). The resultant purified fraction was subjected to dialysis against PBS(-) and a buffer was then exchanged.

### Example 2: Selection of phage binding to monoclonal antibody YSB-2

A phage library having random sequences of 15 amino acid residues was prepared by the method described in Biochemistry, 35, 10441 (1996). Monoclonal antibody YSB-2 was first diluted with PBS(-) in order to immobilize monoclonal antibody YSB-2 on wells of a 96-well microplate (Nunc), 2 µg of the antibody was added per well, and the mixture was allowed to react at 4°C overnight. The plate on which the antibody was immobilized was subjected to blocking using a buffer solution (PBS(-) containing 1% bovine serum albumin and 0.05% Tween 20) at room temperature for one hour. After blocking, about 10¹² virion phage library was added to 100 µl of buffer solution (PBS(-) containing 1% bovine serum albumin and 0.05% Tween 20) and the mixture was allowed to react at room temperature for one hour. The reaction product was washed ten times with a wash (PBS(-) containing 0.05% Tween 20) and non-binding phage was removed therefrom. The bound phage was eluted with a glycine buffer solution (pH 2.2) and neutralized with 1M Tris-HCl (pH 9.5) immediately after the elution. The eluted phage was immediately infected with *Escherichia coli* K91kan, cultured in an LB medium containing tetracycline overnight, and the phage was then amplified. The phage that appeared in the medium supernatant was concentrated by precipitation with polyethylene glycol and this phage was then used in the second panning. This operation was repeated three times in total and the phage binding to YSB-2 was selected.

### Example 3: Selection of YSB-2 binding phage by ELISA

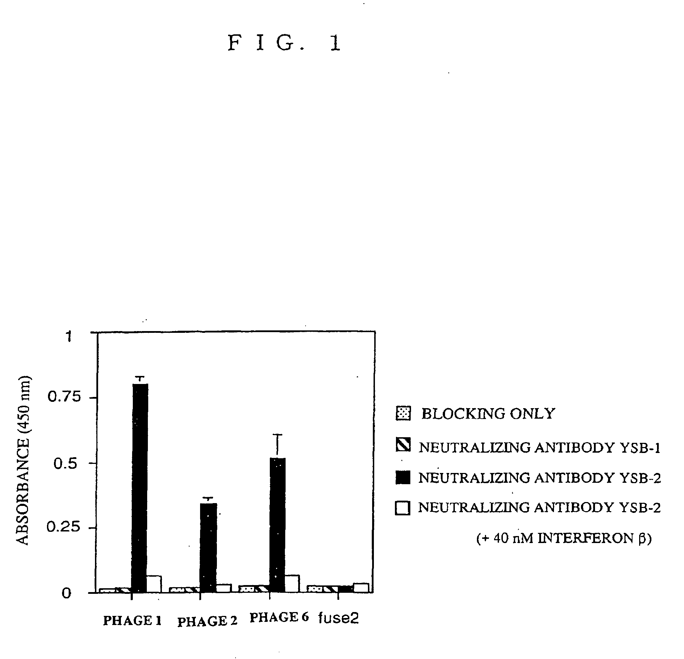

The phage selected in Example 2 was reinfected with *Escherichia coli* K91kan and single colonies were formed on an LB agar medium containing tetracycline. Each colony was cultured in the LB medium containing tetracycline overnight and, on the next day, the phage which appeared in the supernatant was precipitated and purified with polyethylene glycol. The resultant phage solution was added, in an amount of about 10¹⁰ virion, to each well of the 96-well microplate (Example 2) having YSB-2 previously immobilized thereon and allowed to react at room temperature for one hour. After washing four times with a wash (PBS(-) containing 0.05% Tween 20), 5,000 times-diluted horseradish peroxidase labeled anti-M13 phage antibody (Amersham Pharmacia Biotech) was added and the mixture was allowed to react at room temperature for 30 minutes. After washing four times, 3,3',5,5'-tetramethylbenzidine, a substrate, was added and a color was developed at room temperature for 5 minutes. After the reaction was terminated with 1M sulfuric acid, the absorbance at 450 nm was measured using a microplate reader. The results thereof are shown in Fig. 1. The results of ELISA are shown for three types of phages having different sequences. Each phage bound to YSB-2 only, and did not bind to another neutralizing antibody for interferon β, YSB-1 (Sugi, M. et al., Hybridoma, 6, 313-320 (1987)) and a well which was subjected only to blocking with BSA. Further, binding between the phages and YSB-2 was eliminated by the addition of 40 nM interferon β (Feron, Toray Industries, Inc.). Thus, it is considered that each phage recognizes the antigen binding site of YSB-2.

### Example 4: Determination of nucleotide sequence

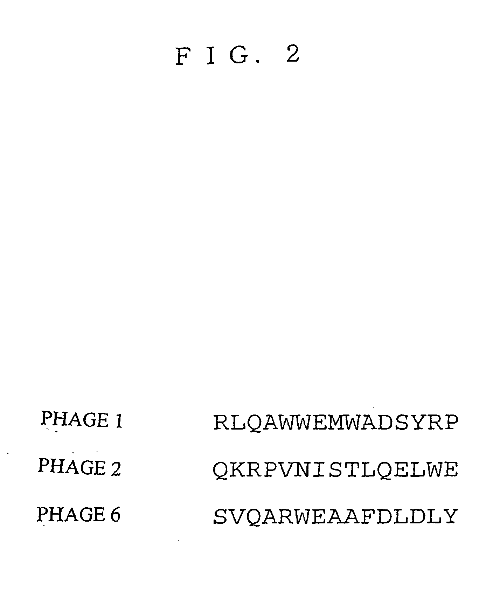

The nucleotide sequence was determined for the phage clones bound to YSB-2 in Example 3. Each phage was treated with phenol and chloroform to be deproteinized. DNA was then purified by ethanol precipitation and employed as a template in the determination of the nucleotide sequences. A primer was set based on the sequence of Fuse5 vector, a vector, and determined by a cycle sequence method. As a result, three types of sequences were confirmed. The amino acid sequences which are deduced from the nucleotide sequences are shown in Fig. 2.

### Example 5: Synthesis of peptide

Based on the amino acid sequences deduced from the nucleotide sequences (see Fig. 2), three types of peptides comprising 15 amino acid residues were synthesized using an automatic peptide synthesizer (TORAY RESEARCH CENTER, Inc.). The synthetic peptides were designated as SYR1, 2, and 6 in accordance with the sequences of phages 1, 2, and 6.

### Example 6: Examination of the antiviral activity of peptide (a bioassay method using FL cells and sindbis viruses)

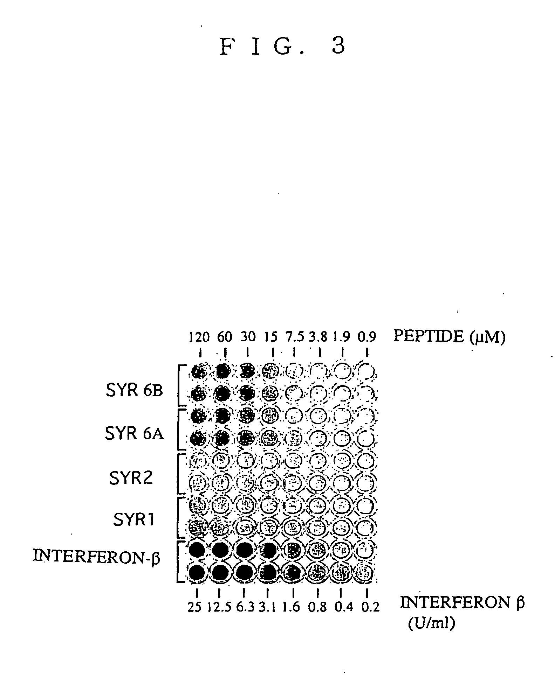

The antiviral activity of the resultant synthetic peptides was inspected by a bioassay method using human amnion-derived FL cells and sindbis viruses (Armstrong, J. A., Methods in Enzymology, 78, 381-387 (1981)). FL cells were inoculated on wells of a 96-well microtiter plate (IWAKI GLASS CO., LTD.) at 3.5 x 10⁵ cells/well and cultured for 24 hours. Thereafter, each peptide was added at the concentration shown in Fig. 3 (0.9 µM to 120 µM) and cultured for 24 hours. As a control, 0.2 U/ml to 25 U/ml of interferon β (Feron, Toray Industries, Inc.) was added and cultured for 24 hours. Thereafter, sindbis viruses were added to each well and cultured for 15 hours. The culture solution was discarded, the plate was immersed in a crystal violet solution containing formalin for about 15 minutes, and living cells were immobilized and stained. As a result, peptides SYR1 and 2 did not exhibit any antiviral activity even with the addition of as much as 120 µM thereof, however, SYR6 began to exhibit the antiviral activity at a concentration of 30 µM (Fig. 3).

### Example 7: Examination of the antiviral activity of peptide SYR6 (a bioassay method using FL cells and vesicular stomatitis viruses (VSV))

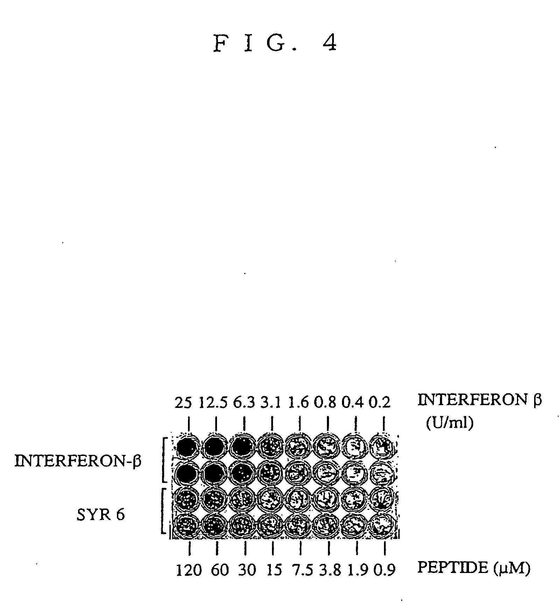

In order to deny the possibility that the antiviral activity of peptide SYR6 obtained in Example 6 is attained by inhibiting the binding and adsorption between FL cells and sindbis viruses, a bioassay was carried out in which the sindbis viruses were replaced with vesicular stomatitis viruses (VSV). The method was in accordance with the method described in Example 6. The results are shown in Fig. 4. In the case where VSV was used also, peptide SYR6 began to exhibit the antiviral activity at a concentration of 30 µM as with the case where sindbis viruses were used.

### Example 8: Examination of the antiviral activity of peptide SYR6 (examination of the antiviral activity in the presence of an interferon neutralizing antibody)

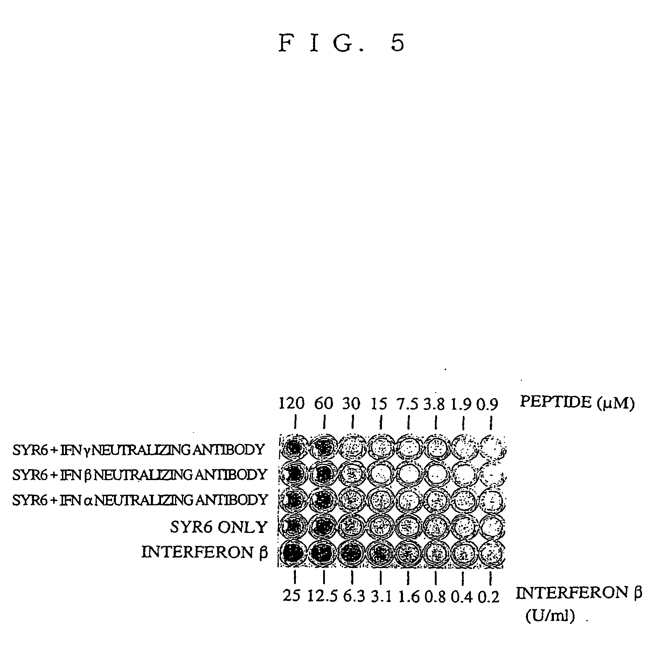

In order to deny the possibility that the antiviral activity of peptide SYR6 is attained by inducing endogenous production of interferon, the antiviral activity in the presence of various interferon neutralizing antibodies was examined. FL cells were inoculated on wells of a 96-well microtiter plate (IWAKI GLASS CO., LTD.) at 3.5 x 10⁵ cells/well and cultured for 24 hours. Thereafter, peptide SYR6 was added at the concentration shown in Fig. 5 (0.9 µM to 120 µM) and cultured for 24 hours. At this time, neutralizing antibody MIF-1 for interferon α at 1.2 µg/well (Hayashibara biochemical laboratories, Inc., catalog No. MIF-1), neutralizing antibody YSB-1 for interferon β at 15.8 µg/well (Sugi, M. et al., Hybridoma, 6, 313-320 (1987)), and a neutralizing antibody for interferon γ at 1 µg/well (Genzyme R & D systems, catalog No. 1598-00) were added and cultured. Thereafter, sindbis viruses were added to each well and cultured for 15 hours. In this case also, various neutralizing antibodies were added and cultured under the same conditions as described above. The culture solution was discarded, the plate was immersed in a crystal violet solution containing formalin for about 15 minutes, and living cells were immobilized and stained. The results are shown in Fig. 5. The antiviral activity of peptide SYR6 was not affected at all by the presence of various interferon neutralizing antibodies. Therefore, the possibility that the antiviral activity of peptide SYR6 was attained by inducing the production of endogenous interferon was denied.

### Example 9: Secondary structure of peptide SYR6

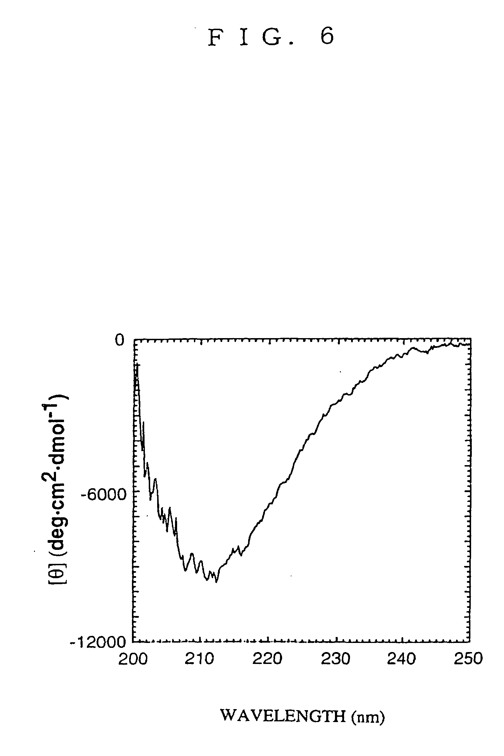

In order to analyze the secondary structure of peptide SYR6, which was clarified as having the antiviral activity, the circular dichroism (CD) was measured. Peptide SYR6 was dissolved in PBS(-) at a concentration of 0.25 mg/ml. A CD spectrum was measured at room temperature (about 24°C) using Jasco J-500A. As the average residue molecular weight of peptide SYR6, 117.47, which was calculated from the amino acid sequence, was used. The results are shown in Fig. 6. As a result of the measurement, the CD spectra of peptide SYR6 exhibited a typical β-sheet structure. The secondary structure was analyzed in accordance with the method by Chen et al. (Chen, Y. H., et al., Biochemistry, 11, 4120-4131 (1972)). As a result, it was found that the secondary structure contained 49% β-sheet structure and 18% α helix structure.

### Example 10: Measurement of antiviral activity of SYR6 peptide substituted with an alanine residue

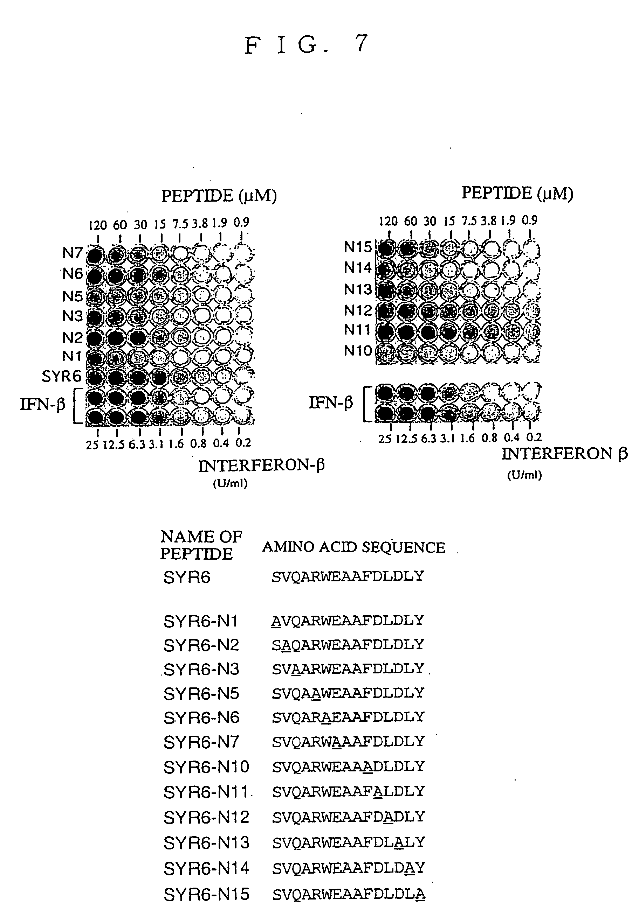

In order to identify an amino acid residue that plays an important role in expressing the antiviral activity of peptide SYR6, 12 types of varied peptides were prepared through chemical synthesis in which each of all the amino acid residues other than alanine of SYR6 were respectively substituted with alanine (peptides were successively designated as N1 to N15 in order from the N-terminus side in accordance with the number of the amino acid residue converted into alanine. Since the fourth, eighth, and ninth residues are alanine, excluding these, a total of 12 types of peptides was prepared). The sequences are shown in the lower part of Fig. 7. The antiviral activities of these peptides were examined by a bioassay method using human amnion-derived FL cells and sindvis viruses shown in Example 6 (Armstrong, J. A., Methods in Enzymology, 78, 381-387 (1981)). FL cells were inoculated on wells of a 96-well microtiter plate (IWAKI GLASS CO., LTD.) at 3.5 x 10⁵ cells/well and cultured for 24 hours. Thereafter, each peptide was added at a concentration of 0.9 µM to 120 µM and cultured for 24 hours. As a control, 0.2 U/ml to 25 U/ml of interferon β (Feron, Toray Industries, Inc.) was added and cultured for 24 hours. Thereafter, sindbis viruses were added to each well and cultured for 15 hours. The culture solution was discarded, the plate was immersed in a crystal violet solution containing formalin for about 15 minutes, and living cells were immobilized and stained. As a result, it was found that N2, N6, N11, and N12 exhibited antiviral activity that is equivalent to SYR6, while the activities of N1, N5, N10, and N14 substantially disappeared. Further, the activities of N3, N7, N13, and N15 were lowered compared to SYR6. It is considered that the amino acid residues with respect to which lowering or disappearance of the activity was exhibited by substitution into alanine play an important role in expressing the antiviral activity of peptide SYR6.

### Example 11: Construction of solid-phase ligand binding assay using a soluble interferon receptor

Total RNA was extracted from 2 x 10⁶ human FL cells using Isogen (Nippon Gene Co. Ltd.). 1 µg of total RNA was isolated from genes existing in the extracellular region of interferon receptors AR1 and AR2 strands by PCR using the following primers.
- AR1 sense: 5'-ggg gaa ttc gta act ggt ggg atc tgc ggc-3'
- AR1 antisense: 5'-ccc gga tcc tta gag gta ttt cct ggt tt-3'
- AR2 sense: 5'-ggg gaa ttc gag aag act cta aaa ata gc-3'
- AR2 antisense: 5'-ccc gga tcc ttg gca gat tct gct gat tc-3'

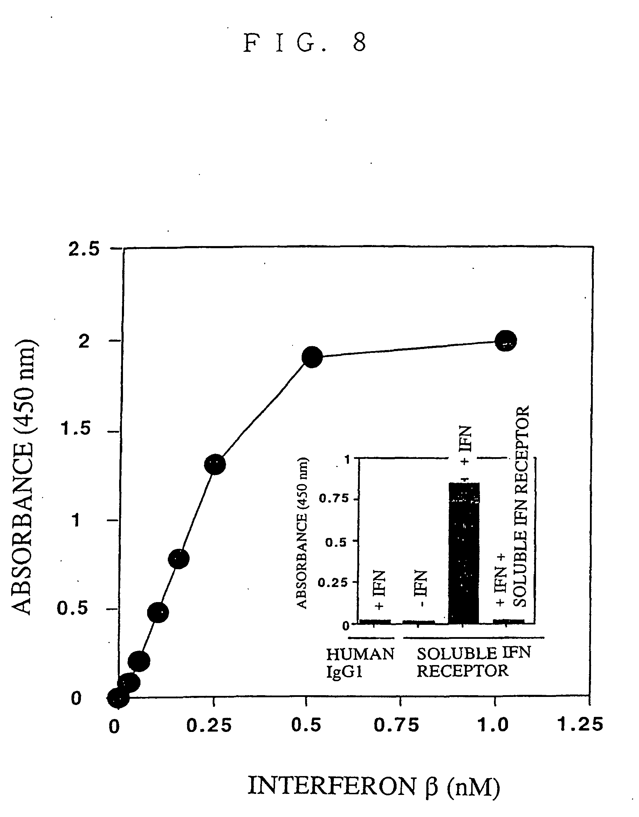

AR1 is designed to encode the extracellular region of amino acid residues 1 to 436 (Uze, G. et al., Cell, 60, 225-234 (1990)) and AR2 is designed to encode the extracellular region of amino acid residues 1 to 243 (Domanski, P. et al., J. Biol. Chem., 270, 21606-21611 (1995)). The resultant PCR fragment was connected to EcoRI site and BamHI site of vector HuIgG1/SRα for expressing a fusion protein with a human IgG1Fc region. The constructed expression vector was subjected to co-transfection with COS-1 cell by the DEAE-Dextran method, thereby transiently expressing a protein. The soluble interferon receptor, produced in the culture solution, was purified using Protein A Sepharose CL-4B column (Amersham Pharmacia Biotech). The purified soluble interferon receptor (1 µg/ml) was allowed to react on a 96-well microplate (Maxisorp, Nunc) at 100 µl/well at 4°C overnight to be immobilized on the microplate. After blocking with PBS(-) containing 1% BSA and 0.05% Tween 20, human interferon β (Feron) was added so as to bring the amount from 25 pM to 1 nM, and this was shaken at room temperature for 2 hours. After washing four times with PBS(-) containing 0.05% Tween 20, 50 µl of horseradish peroxidase labeled anti-IFN-β antibody YSB-1 (Yamazaki, S. et al., J. Immunoassay, 10, 57-73 (1989)) was added and the mixture was allowed to react at room temperature for one hour. After washing four times with PBS(-) containing 0.05% Tween 20, 100 µl/well of TMB substrate (DAKO) was added and the reaction was carried out at room temperature for two minutes to develop a color. Thus, the absorbance at 450 nm was measured. In this system, the binding between human interferon β and a soluble interferon receptor was observed in a concentration-dependent manner (Fig. 8). Human interferon β did not bind to a control human IgG1. Further, when human interferon β was not added to this system, a signal was not detected. The signal obtained when 0.5 nM human interferon β was added was completely eliminated by the addition of 27 nM soluble interferon receptor. Thus, it was confirmed that this solid-phase ligand binding assay was a system for detecting a specific ligand-receptor binding. In this system, 25 pM human interferon β (22 pg, about 4.4 U) was detectable.

### Example 12: Analysis of binding of peptides SYR1, 2, and 6 to a soluble interferon receptor using a competitive binding assay

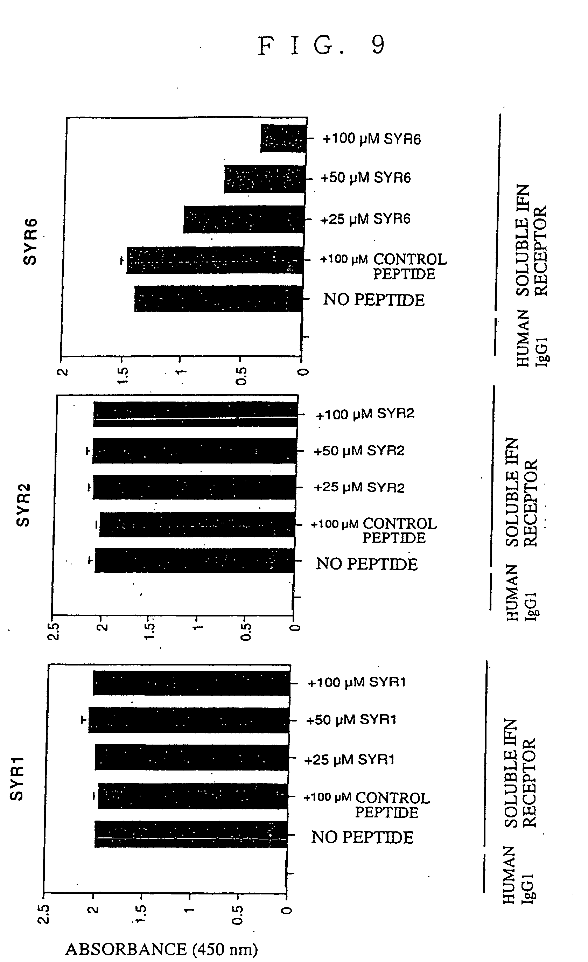

Using the solid-phase ligand binding assay constructed in Example 11, examination was conducted regarding whether or not peptides SYR1, 2, and 6 were capable of inhibiting binding between human interferon β and a soluble interferon receptor. As a result, it was found that SYR1 and SYR2 did not inhibit the binding even with the addition of 100 µM thereof, while SYR6 inhibited the binding from 25 µM to 100 µM in a concentration-dependent manner (Fig. 9). The above results suggest that SYR6 mimics human interferon β and expresses the antiviral activity through an interferon receptor.

### Example 13: Analysis of molecular weight of peptide SYR6 in a solution using an ultracentrifuge for analysis

The molecular weight of peptide SYR6 in a solution was determined by a sedimentation equilibrium method using an ultracentrifuge for analysis. Peptide SYR6 was suspended in PBS(-) so as to bring the concentration to 140 µM. This sample was centrifuged at a rotational speed shown in the table below at 4°C for about 20 hours. Thus, the sedimentation equilibrium was prepared. As a partial specific volume of the peptide, 0.72 ml/g, which is an average value of a partial specific volume of each amino acid, was used. The results are shown in the table. The average molecular weight of peptide SYR6 in a solution was calculated at 1,562 ± 49.2, which was substantially congruous with the molecular weight calculated from the amino acid sequence, i.e., 1,784. Therefore, it is considered that peptide SYR6 exists as a monomer in a solution and expresses an antiviral activity.

| Table: Average molecular weight of peptide SYR6 in a solution | | | |
|---|---|---|---|
| Peptide | Rotor speed (rpm) | Molecular weight | Average molecular weight |
| SYR-6 | 40,000 | 1,643 | 1,562 ± 49.2 |
| | 50,000 | 1,473 | |
| | 60,000 | 1,569 | |

All publications, patents and patent applications cited herein are incorporated herein by reference in their entirety.

### INDUSTRIAL APPLICABILITY

The present invention provides a low molecular weight peptide mimicking a cytokine and a method for newly producing the same.

### SEQUENCE LISTINGS FREE TEST

SEQ ID NO: 1: a peptide that is synthesized based on the DNA sequence of phage binding to monoclonal antibody YSB-2

## Claims

1. A method for screening a cytokine-like peptide, comprising searching for a peptide which binds to an antibody having an activity of neutralizing a cytokine and which is capable of expressing a biological activity of the cytokine from among peptides not confirmed as having the biological activity of the cytokine.

2. The screening method according to claim 1, wherein the cytokine is an interferon.

3. A cytokine-like peptide, which is obtained by the screening method according to claim 1.

4. A cytokine-like peptide, which contains an amino acid sequence derived from the amino acid sequence of the cytokine-like peptide according to claim 3 by deletion, substitution, insertion or addition, or modification of at least one amino acid residue and which is capable of expressing the biological activity of the cytokine.

5. The cytokine-like peptide according to claim 4, wherein the amino acid sequence of the cytokine-like peptide is the amino acid sequence shown in SEQ ID NO: 1 of the Sequence Listing and the cytokine is an interferon.

6. A cytokine-like peptide capable of expressing a biological activity of an interferon, wherein the amino acid sequence of the cytokine-like peptide contains the amino acid sequence shown in SEQ ID NO: 1 of the Sequence Listing.

7. A method for producing a cytokine-like peptide, comprising obtaining a peptide capable of expressing a biological activity of a cytokine, wherein the cytokine-like peptide obtained by the screening method according to claim 1 is employed as a leading compound and deletion, substitution, insertion or addition, or modification of at least one amino acid residue is applied thereto.

8. A medicine comprising, as an active ingredient, the cytokine-like peptide according to claim 3.

9. A medicine comprising, as an active ingredient, the cytokine-like peptide according to claim 4.

10. A medicine comprising, as an active ingredient, the cytokine-like peptide according to claim 6.
